Europäisches Patentamt

(19) European Patent Office

Office européen des brevets

(11) Veröffentlichungsnummer: **0 147 707**
**B1**

(12) **EUROPÄISCHE PATENTSCHRIFT**

(45) Veröffentlichungstag der Patentschrift:
18.03.87

(51) Int. Cl.⁴: **C 07 C 121/66,** C 07 B 57/00,
A 61 K 31/275

(21) Anmeldenummer: 84115038.6

(22) Anmeldetag: 10.12.84

(54) 1,7-Diphenyl-3-methylaza-7-cyan-8-methyl-nonan zur Verwendung bei der Bekämpfung von Krankheiten.

(30) Priorität: 10.12.83 DE 3344755

(43) Veröffentlichungstag der Anmeldung:
10.07.85 Patentblatt 85/28

(45) Bekanntmachung des Hinweises auf die Patenterteilung:
18.03.87 Patentblatt 87/12

(84) Benannte Vertragsstaaten:
AT BE CH DE FR GB IT LI LU NL SE

(56) Entgegenhaltungen:
EP-A-0 029 175
EP-A-0 064 158
EP-A-0 066 246
FR-A-2 116 564
FR-A-2 116 565

ARZNEIMITTEL-FORSCHUNG, Band 31, Nr. 5, Mai
1981, Seiten 773-780 R. MANNHOLD et al.: "The
influence of aromatic substitution on the negative
inotropic action of Verapamil 1,2) in the isolated cat
papillary muscle"
CHEMICAL ABSTRACTS, Band 97, Nr. 1, 5. July
1982, Seite 29, Zusammenfassung 299g Columbus,
Ohio, US H.D. HOELTJE: "Theoretical study on
structure-activity relations for ring-substituted
verapamil derivatives"
Arch. Pharm. 1982, 315(4), 317-323

(73) Patentinhaber: BASF Aktiengesellschaft, Carl-
Bosch- Strasse 38, D-6700 Ludwigshafen (DE)

(72) Erfinder: Raschack, Manfred, Dr.,
Donnersbergstrasse 7, D-6714 Weisenheim am
Sand (DE)
Erfinder: Kreiskott, Horst, Prof. Dr.
Pharmakologe, Am Boehlig 15, D-6706
Wachenheim (DE)
Erfinder: Seitz, Werner, Dr. Chemiker,
Bismarckstrasse 22 b, D-6831 Plankstadt (DE)

(56) Entgegenhaltungen: (Fortsetzung)
Die Akte enthält technische Angaben, die nach
dem Eingang der Anmeldung eingereicht wurden
und die nicht in dieser Patentschrift enthalten sind.

**Beschreibung**

Phenylacetonitrile sind bereits bekannt (DE-PS 1 154 810). So werden Verapamil und Gallopamil als antiarrhythmika bzw. Koronarmittel eingesetzt (vgl. Rote Liste 1983).

Es ist weiter beschrieben worden, daß der Austausch der Methoxygruppen an den Phenylkernen in diesen Verbindungen gegen Wasserstoffatome zu unbrauchbaren Verbindungen führt (Arzneim.-Forsch. 31, 773 (1981), auch Pharm. 1982, 315(4), 317-323).

Es wurde nun gefunden, daß sich das 1,7-Diphenyl-3-methylaza-7-cyan-8-methyl-nonan und dessen Salze mit physiologisch verträglichen Säuren sehr gut zur protektiven Behandlung von hypoxischen Gewebsschädigungen eignet.

Das 1,7-Diphenyl-3-methylaza-7-cyan-8-methyl-nonan kann auch in Form seiner optischen Antipoden eingesetzt werden, die man erhält, indem man das Racemat mit chiralen Säuren umsetzt, das so erhaltene Gemisch der diastereomeren Salze trennt, aus den so erhaltenen Salzen die Basen freisetzt und diese gegebenenfalls in ihre Salze mit physiologisch verträglichen Säuren überführt.

Beispiele für chirale Säuren sind die optisch aktiven Formen der Campher-10-sulfonsäure, alpha-Bromcampher-pi-sulfonsäure, Camphersäure, Menthoxyessigsäure, Weinsäure, Mandelsäure, alpha-Methoxyalpha-trifluormethyl-phenylessigsäure, 0,0-Diacetyl-, 0,0-Dibenzoyl- und 0,0-Di-4-toluoyl-weinsäure.

Die Trennung der diastereomeren Salze kann in üblicher Weise, z.B. durch fraktionierte Kristallisation oder Säulenchromatographie, erfolgen.

Die Racemattrennung kann auch säulenchromatographisch erfolgen. Als chirale Trägermaterialien kommen dafür beispielsweise in Betracht. optisch aktive Polymethylacrylate; 2,4-Dinitrobenzoylphenylglycin gebunden an Aminopropylkieselgel; acetylierte und/oder benzoylierte quervernetzte Cellulose; Cellulose-tribenzylether und/oder Cellulosephenylcarbamate. In diesem Fall erhält man die reinen antipoden direkt, indem man das Racemat über eine Säule aus den genannten Trägermaterialien führt.

Als physiologisch verträgliche Säuren, die sich zur Salzbildung mit dem 1,7-Diphenyl-3-methylaza-7-cyan-8-methyl-nonan und dessen Antipoden eignen, seien speziell Schwefelsäure, Phosphorsäure, Weinsäure, Essigsäure, Bernsteinsäure, Milchsäure, Zitronensäure, Amidosulfonsäure und insbesondere Salzsäure genannt.

Die Herstellung des 1,7-Diphenyl-3-methylaza-8-methyl-nonans erfolgt in an sich bekannter Weise, z.B. durch Umsetzen von alpha-Isopropylbenzylcyanid mit N-(3-Chlorpropyl)-N-methylphenethylamin.

Wie bereits erwähnt, eignet sich das 1,7-Diphenyl-3-methylaza-8-methyl-nonan sehr gut zur protektiven Behandlung vom hypoxischen Gewebsschädigungen. Solche Schäden treten bei Sauerstoffmangel insbesondere am Herzen, am Gehirn und an der Niere bei Ischämie- oder Schockzuständen rasch ein, verursachen oft bleibende Defekte oder führen sogar zum Tod. Auch bei Durchblutungsdrosselung von Organen, die z.B. wegen operativer Eingriffe vorgenommen werden muß, sind Hypoxie-Schäden gefürchtet.

Die gute Wirkung des 1,7-Diphenyl-3-methylaza-8-methyl-nonans (im folgenden mit "Ä" bezeichnet) ließ sich in folgenden Versuchen zeigen:

Männliche Wistar-Ratten Don 250 bis 300 g Körpergewicht wurden in Thiobutabarbitalnarkose (100 mg/kg KG i.p.) mit einem Sauerstoffmangelgemisch standardisiert beatmet (2 % $O_2$, 98 % $N_2$, 7 min). Unter Anwendung der Gefrierstopptechnik wurde die Herzspitze entnommen und nach mechanischer Zerkleinerung unter Zusatz von 0,6 N Perchlorsäure aufgeschlossen. Nach Zentrifugieren wurden im überstand Kreatinphosphat (nach Bergmeyer, H.U.: Methoden der enzymatischen Analyse, Verlag Chemie, Weinheim, 2, 1825 (1974)), Adenosintriphosphat (in Amlehnung an Biochem. Biophys. Acta (Amst.) 1, 292 (1947)) und Glykogen (nach Biochem. J. 56, 1825 (1954)) bestimmt.

Die Prüfsubstanzen wurden zu unterschiedlichen Zeitpunkten vor der Narkose und vor der folgenden Sauerstoffmangelbeatmung den wachen Tieren appliziert. Bestimmt wurde der prozentuale Unterschied der myokardialen Konzentrationen von Adenosintriphosphat (ATP), Creatinphosphat (CP) umd Glykogen bei mit Prüfsubstanz vorbehandelten Tieren im Vergleich zu unbehandelten Hypoxie-Kontrolltieren.

Tabelle 1 zeigt, daß bereits die niedrigste geprüfte Dosis von 5 mg/kg A p.o. die hypoxische Verarmung des Myokards an Creatinphosphat, Adenosintriphosphat und Glykogen deutlich hemmt. Bei Dosissteigerung nimmt die Schutzwirkung zu.

**Tabelle 1**

Myokardprotektion 6 h nach oraler Applikation von A an der Ratte

| Applizierte Menge A mg/kg | prozentualer Unterschied der Menge an | | |
|---|---|---|---|
| | CP | ATP | Glykogen |
| 5 | 29 | 24 | 10 |
| 10 | 47 | 37 | 27 |
| 20 | 80 | 63 | 50 |

Tabelle 2 zeigt die Langzeit-Schutzwirkung von A, die bereits 2 h nach Applikation der gut verträglichen Dosis von 20 mg/kg für die Parameter CP und ATP deutlich nachweisbar ist und bis zu 24 h anhält.

**Tabelle 2**

Kardioprotektive Langzeitwirkung von A an der Ratte Dosis: 20 mg/kg p.o.

| h nach Substanzgabe | prozentualer Unterschied der Menge an | | |
|---|---|---|---|
| | CP | ATP | Glykogen |
| 2 | 41 | 31 | -1 |
| 6 | 80 | 63 | 50 |
| 12 | 42 | 42 | 19 |
| 18 | 32 | 40 | 16 |
| 24 | 22 | 11 | 26 |

Tabelle 3 belegt, daß A dem Verapamil in der kardioprotektiven Wirkung deutlich überlegen ist. Im Unterschied zu A hat Verapamil bei gleicher Dosierung und selbst in 4fach höherer Dosis 6 h nach oraler Gabe keine nennenswerte Schutzwirkung gegen den durch Hypoxie ausgelösten Abfall der myokardialen Spiegel von CP, ATP und Glykogen.

**Tabelle 3**

Vergleich der Myokardprotektion durch A und Verapamil 6 h nach oraler Applikation an der Ratte

| Substanz | Dosis mg/kg | prozentualer Unterschied der Menge an | | |
|---|---|---|---|---|
| | | CP | ATP | Glykogen |
| A | 20 | 80 | 63 | 50 |
| Verapamil | 20 | -9 | 0 | 9 |
| " | 40 | 9 | 9 | -5 |
| " | 80 | 21 | 20 | 2 |

Tabelle 4 enthält einen Wirksamkeitsvergleich für das Razemat und die optischen Isomeren von A. Beide Enantiomeren wirken bei separater Prüfung dosisabhängig kardioprotektiv. Die Wirkdosis der linksdrehenden Form liegt erheblich unter der des rechtsdrehenden Antipoden. Im Vergleich zum Razemat ist (-) A etwa doppelt so wirksam, während (+) A etwa ein Viertel der Wirksamkeit des Razemats aufweist.

**Tabelle 4**

Myokardprotektion an der Ratte 6 h nach oraler Applikation Die $ED_{50}$ ist die Prüfsubstanz-Vorbehandlungsdosis, die um 50 % höhere ATP-Spiegel bewirkt als bei der Hypoxie-Kontrollgruppe

| Substanz | $ED_{50}$ mg/kg | Relative Wirkstärke |
|---|---|---|
| (±) A | 13.6 | 1 |
| (-) A | 6.6 | 2.06 |
| (+) A | 50.3 | 0.27 |

Überraschenderweise wurde weiter gefunden, daß A selbst in der Dosis von 20 mg/kg p.o., die an der Ratte maximale kardioprotektive Wirkung besitzt, keinen nennenswerten Einfluß auf Blutdruck (gemessen mit Piezo-Pulsaufnehmern) und Herzfrequenz von spontan hypertensiven Ratten hat (Tabelle 5); die Veränderungen sind nicht signifikant. Somit besitzt A überraschenderweise gute kardioprotektive Eigenschaften (vgl. Tab. 2 bis 4) in hämodynamisch neutralen Dosen. Demgegenüber führt Verapamil in vergleichbaren Dosen zu den bekannten Effekten auf Herz und Kreislauf, wie Blutdrucksenkung und A.V.-Überleitungshemmung.

**Tabelle 5**

Einfluß von A auf Blutdruck und Herzfrequenz wacher spontan hypertensiver Ratten
Dosis: 20 mg/kg p.o.

|  | Ausgangswert | 6 h nach Substanzgabe | Änderung in % |
|---|---|---|---|
| Blutdruck | 229 | 217 | -5 |
| Herzfrequenz | 398 | 378 | -5 |

Somit ist bei A auch die Gefahr der Provokation von Erregungsleitungsstörungen erheblich geringer als bei Verapamil. Der Einfluß auf die AV-Überleitung wurde an einer dafür geeigneten Spezies, dem Hund, geprüft. Selbst das 5fache derjenigen Dosis, die bei Verapamil (0,4 mg/kg i.v.) bei allen Versuchstieren zum Auftreten von AV-Blocks II. Grades sowie zu starker Verlängerung der PQ-Dauer führt, ist im Fall von A völlig frei von Beeinträchtigungen der AV-Überleitung. Somit besitzt A, unter Berücksichtigung der überlegenen kardioprotektiven Wirkung, eine erheblich größere therapeutische Breite als Verapamil.

Auch in einer weiteren Versuchsanordnung läßt sich die gute Verträglichkeit von A gegenüber Verapamil zeigen: Verapamil bewirkt am narkotisierten Schwein in der Dosis 0,2 mg/kg i.v. die erwartete starke Zunahme der PQ-Dauer sowie Abnahme der Herzkontraktilität (maximale systolische Druckanstiegsgeschwindigkeit im linken Ventrikel). Demgegenüber hat die Injektion einer 20fach höheren Dosis von A (4 mg/kg i.v.) keine nennenswerten Änderungen dieser Parameter zur Folge,

Eine im Vergleich zu Verapamil überlegene Schutzwirkung gegen zerebrale Hypoxie ließ sich auch an der Maus zeigen: Weibliche Mäuse (NMRI-Ivanovas, Gewicht 24 bis 26 g) wurden zur Prüfung der Hypoxietoleranz einzeln in je ein Glasrohr gebracht, das über ein Rotameter mit einem Gasgemisch aus 96,5 % $N_2$ und 3,5 % $O_2$ mit einer Strömungsgeschwindigkeit von 4 l/min durchströmt wurde. Die Zeitmessung bis zum Exitus begann mit dem Einsetzen des Gasgemischdurchflusses. Im Gegensatz zu der in dieser Versuchsanordnung unwirksamen Vergleichsverbindung Verapamil bewirken A und seine Antipoden (besonders (-) A) dosisabhängig eine Erhöhung der Zahl der Versuchstiere, die nach 3 min überleben. Alle Kontrolltiere sind - ebenso wie die mit Verapamil vorbehandelten Tiere - nach 3 min gestorben.

**Tabelle 6**

Hypoxietoleranz an der Maus, Prüfung 1 h nach oraler Verabfolgung der Versuchssubstamzen.
$ED_{50}$ bedeutet, daß bei der angegebenen Dosis 50 % der Tiere eine Mamgelbeatmung von 3 min überlebten.

| Substanz | $ED_{50}$ mg/kg |
|---|---|
| A | 88 |
| (-) A | 48 |
| (+) A | 100 |

A zeichnet sich somit durch eine hohe orale Verfügbarkeit und lange Wirkdauer aus. Darüber hinaus ist es besonders gut verträglich und führt nicht zur Blutdrucksenkung. Die Selektivität der organprotektiven Wirkung von A beinhaltet den Vorzug größerer therapeutischer Sicherheit, da die Substanz auch bei Patienten mit Dorgeschädigter AV-Überleitung oder labilem Blutdruck gefahrlos eingesetzt werden kann.

Insbesondere eignen sich A und seine Antipoden zur Behandlung folgender Indikationen:

Sauerstoffmangelzustände vitaler Organe, wie Herz, Gehirn und Niere, infolge von Durchblutungsstörungem oder Blutverlust (hämorrhagischer Schock), hypobare und funktionelle Hypoxiezustände (Höhenkrankheit, Epilepsie und Herzarrhythmie); Intoxikationen; traumatische Organschädigungen; Organprotektion (bei Herz-, Hirn- und Nierenoperationen) sowie bei Transplantationen.

Die erfindungsgemäße Verbindung und ihre Antipoden können in üblicher Weise oral oder parenteral (intravenös, intramuskulär, intraperitoneal) verabfolgt werden.

Die Dosierung hängt von alter, Zustand und Gewicht des Patienten sowie von der Applikationsart ab. In der Regel beträgt die tägliche Wirkstoffdosis zwischen etwa 1 und 10 mg/kg Körpergewicht bei oraler Gabe und zwischem etwa 0,1 und 1 mg/kg Körpergewicht bei parenteraler Gabe.

Die erfindungsgemäße Verbindung und ihre Antipoden können in den gebräuchlichen galenischem Applikationsformen fest oder flüssig angewendet werden, z.B. als Tabletten, Filmtabletten, Kapseln, Pulver, Granulate, Dragees oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1975). Die so erhaltenen Applikationsformen enthalten den Wirkstoff normalerweise in einer Menge von 0,1 bis 99 Gewichtsprozent.

Die folgenden Beispiele erläutern die Erfindung.

### Beispiel 1

( ± )-1,7-Diphenyl-3-methylaza-7-cyan-8-methyl-nonan-hydrochlorid

In einem Dreihalskolben, der mit Rührwerk, Tropftrichter und Rückflußkühler ausgerüstet war, wurden 15,9 g (0,1 mol) alpha-Isopropylbenzylcyanid in 20 ml Toluol gelöst. Zu dieser Lösung gab man 29,5 g 85 %iges technisches Kaliumhydroxidpulver und 0,3 g Tetrabutylammoniumiodid. Unter kräftigem Rühren wurde das Reaktionsgemisch auf 80°C erwärmt und bei dieser Temperatur beginnemd eine Lösung von 21,2 g (0,1 Mol) N-(3-Chlor-propyl)-N-methylphenethylamin in 20 ml Toluol in dem Maße zugetropft, daß eine Reaktionstemperatur von 85°C nicht überschritten wurde. Nach beendeter Zugabe wurde 3 h bei 85 bis 90°C nachgerührt. Die erkaltete Reaktionsmischung wurde mit 100 ml Wasser und 100 ml Toluol versetzt, die Toluolphase abgetrennt und mehrmals mit Wasser gewaschem. Nach Trocknen und Abziehen des Lösungsmittels wurden 30 g Rückstand erhalten, die in 200 ml Essigester gelöst und anschließend mit ethanolischer Salzsäure versetzt wurde. Nach 15 h wurden 32 g (85 %) Hydrochlorid isoliert, Fp. 166 - 168°C.

### Beispiel 2

(+)-1,7-Diphenyl-3-methylaza-7-cyan-8-methyl-nonan

40,4 g (0,12 Mol) racemisches 1,7-Diphenyl-3-methylaza-7-cyan-8-methyl-nonan und 48,5 g (0,12 Mol) (-)-0,0'-Di-4-toluoyl-L-weinsäure wurden unter Erwärmen in 400 ml Isopropamol gelöst. Das über Nacht ausgefallene Kristallisat wurde abgesaugt und zweimal aus einem Ethanol-Wasser-Gemisch (4:1) umkristallisiert. Der gefundene Drehwert von $[alpha]_D^{20}$ = - 70,5 (Methanol, c = 10 mg/ml) veränderte sich durch nochmalige Kristallisation nicht. Die aus dem Salz freigesetzte Base zeigt einen Drehwert von $[alpha]_D^{20}$ = + 20,4° (Benzol, c = 10 mg/ml).

Die Base wurde in 100 ml Essigsäureethylester gelöst und mit ethanolischer Salzsäure versetzt, bis der pH-Wert 3 betrug. Nach Kristallisation aus Essigsäureethylester/Ethanol (4:1) und Trocknen erhielt man 15,6 g (70 %) des Hydrochlorids, Fp = 184-185°C, $[alpha]_D^{20}$ = + 11,3° (Ethanol, c = 10 mg/ml).

### Beispiel 3

(-)-1,7-Diphenyl-3-methylaza-7-cyan-8-methyl-nonan

Die nach Beispiel 2 anfallende Mutterlauge der Ausfällung nit (-)-0,0'-Di-4-toluoyl-L-weinsäure wurde in Vakuum eingeengt, der Rückstand in Wasser aufgenommen und daraus durch Zugabe von Kaliumcarbonat die Base freigesetzt. Nach Extraktion mit n-Hexan, Trocknen über Magnesiumsulfat und abdestillieren des Lösungsmittels wurden 20,4 g Öl isoliert.

Dieser Rückstand und 24,2 (0,06 Mol) (+)-0,0'-Di-4-toluoyl-D-weinsäure wurden unter Erwärmen in 200 ml Isopropanol gelöst. Nach 3 h wurde das ausgefallene Kristallisat abgesaugt und zweimal aus einem Ethanol-Wasser-Gemisch (4:1) umkristallisiert. Der Drehwert des Salzes $[alpha]_D^{20}$ = + 70,4° (Methanol, c = 10 mg/ml) veränderte sich durch nochmalige Kristallisation nicht. Die aus dem Salz freigesetzte Base zeigte einen Drehwert von $[alpha]_D^{20}$ = - 20,4° (Benzol, c = 10 mg/ml).

Aus der Base wurde wie in Beispiel 2 beschrieben, das Hydrochlorid erhalten. Die ausbeute betrug 16,8 g (75 %), Fp = 184-185°C $[alpha]_D^{20}$ = - 11,3° (Ethanol, c = 10 mg/ml).

**Beispiel 4**

Auf einer Tablettenpresse werden in üblicher Weise Tabletten folgender Zusammensetzung gepreßt:
40 mg Substanz des Beispiels 1
120 mg Maisstärke
13,5 mg Gelatine
45 mg Milchzucker
2,25 mg Aerosil ) (chemisch reine Kieselsäure in submikroskopisch feiner Verteilung)
6,75 mg Kartoffelstärke (als 6 %iger Kleister)

**Beispiel 5**

In üblicher weise werden Dragees folgender Zusammensetzung hergestellt:
20 mg Substanz des Beispiels 3
60 mg Kernmasse
60 mg Verzuckerungsmasse
Die Kernmasse besteht aus 9 Teilen Maisstärke, 3 Teilen Milchzucker und 1 Teil Luviskol (R) VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

**Beispiel 6**

10 g Substanz des Beispiels 3 werden in 5000 ml wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

**Patentansprüche** für die Vertragsstaaten BE, CH, DE, FR, GB, IT, LI, LU, NL, SE
1. ($\pm$)-1,7-Diphenyl-3-methylaza-7-cyan-8-methyl-nonan sowie dessen Antipoden und deren Salze mit physiologisch verträglichen Säuren zur Verwendung bei der Bekämpfung von Krankheiten.
2. Verwendung von ($\pm$)-1,7-Diphenyl-3-methylaza-7-cyan-8-methyl-nonan sowie dessen Antipoden und deren Salze mit physiologisch verträglichen Säuren zur Herstellung von Arzneimitteln zur Bekämpfung hypoxischer Zustände.
3. (+)-1,7-Diphenyl-3-methylaza-7-cyan-8-methyl-nonan.
4. (-)-1,T-Diphenyl-3-methylaza-7-cyan-8-methyl-nonan.
5. Verfahren zur Herstellung der optischen Antipoden des (+)-1,7-Diphenyl-3-methylaza-7-cyan-7-methyl-nonans, dadurch gekennzeichnet, daß man das Racemat der Verbindung mit chiralen Säuren umsetzt, das soerhaltene Gemisch der diastereomeren Salze trennt, aus den so erhaltenen Salzen die Basen treisetzt und diese gegebenenfalls in ihre Salze mit physiologisch verträglichen Säuren überführt.

**Patentansprüche** für den Vertragsstaat AT

Verfahren zur Herstellung der optischen Antipoden des ($\pm$)-1,7-Diphenyl-3-methylaza-7-cyan-7-methyl-nonans, dadurch gekennzeichnet, daß man das Racemat der Verbindung mit chiralen Säuren umsetzt, das so erhaltene Gemisch der diastereomeren Salze trennt, aus den so erhaltenen Salzen die Basen freisetzt und diese gegebenenfalls in ihre Salze mit physiologisch verträglichen Säuren überführt.

**Revendications** pour les Etats contractants: BE, CH, DE, FR, GB, IT, LI, LU, NL, SE
1. ($\pm$)-1,7-diphényl-3-méthylaza-7-cyano-8-méthyl-nonane ainsi que ses antipodes et leurs sels d'acides physiologiquement tolérables, pour l'utilisation dans la lutte contre les maladies.
2. Utilisation de ($\pm$)-1,7-diphényl-3-méthylaza-7-cyano-8-méthyl-nonane ainsi que ses antipodes et leurs sels d'acides physiologiquement tolérebles, pour le préparation de médicaments pour la lutte contre les états hypoxiqnes.

3. (+)-1,7-diphényl-3-méthylaza-7-cyano-8-méthyl-nonane.

4. (-)-1,7-diphényl-3-méthylaza-7-cyano-8-méthyl-nonane.

5. Procédé de préparation des antipodes optiques de la (±)-1,7-diphényl-3-méthylaza-7-cyano-8-méthyl-nonane, caractérisé par le fait qu'on fait réagir la racémate du composé avec des acides chiraux, on sépare le mélange ainsi obtenu des sels diastéréomères, on libère, de ses sels ainsi obtenus, les bases que l'on transforme éventuellement en leurs sels avec des acides physiologiquement acceptables.

**Revendication** pour l'Etat contractant: AT

Procédé de préparation des antipodes optiqnes de la (±)-1,7-diphényl-3-méthylaza-7-cyano-8-méthyl-nonane, caractérisé par le fait qu'on fait réagir le racémate du composé avec des acides chiraux, on sépare le mélange ainsi obtenu des sels diastéréomères, on libère, de ses sels ainsi obtenus, les bases que l'on transforme éventuellement en leurs sels avec des acides physiologiquement acceptables.

**Claims** for the Contracting states: BE, CH, DE, FR, GB, IT, LI, LU, NL, SE

l. (±)-1,7-Diphenyl-3-methylaza-7-cyano-8-methylnonane as well as its antipodes and their salts with physiologically tolerated acids for use in the treatment of disorders.

2. The use of (±)-1,7-diphenyl-3-methylaza-7-cyano-8-methyl-nonane as well as its antipodes and their salts with physiologically tolerated acids for the production of drugs for treating hypoxic conditions.

3. (+)-1,7-Diphenyl-3-methylaza-7-cyano-8-methylnonane.

4. (-)-1,7-Diphenyl-3-methylaza-7-cyano-8-methylnonane.

5. A process for the preparation of the optical antipodes of (±)-1,7-diphenyl-3-methylaza-7-cyano-8-methylnonane, wherein the racemate of the compound is reacted with chiral acids, the resulting mixture of diastereomeric salts is separated, the bases are liberated from the salts thus obtained and, if desired, these bases are converted into their salts with physiologically tolerated acids.

**Claim** for the Contracting state: AT

A process for the preparation of the optical antipodes of (±)-1,7-diphenyl-3-methylaza-7-cyano-8-methylnonane, wherein the racemate of the compound is reacted with chiral acids, the resulting mixture of diastereomeric salts is separated, the bases are liberated from the salts thus obtained and, if desired, these bases are converted into their salts with physiologically tolerated acids.